# EUROPEAN PATENT SPECIFICATION

(11) **EP 4 321 135 B1**
(45) Date of publication and mention of the grant of the patent: **01.01.2025**
(21) Application number: 23209463.1
(22) Date of filing: 09.11.2020
(51) Int. Cl.: A61F 5/44, A61M 25/00, A61F 5/441

(54) **EASY EMPTY URINE COLLECTION BAGS**
LEICHT ENTLEERBARE URINSAMMELBEUTEL
SACS DE COLLECTE D'URINE FACILE À VIDER

(30) Priority: 25.11.2019 US 201962939848 P
(43) Date of publication of application: 14.02.2024
(62) Divisional of application: 20820602.9
(73) Proprietor: Hollister Incorporated, Libertyville, IL 60048-3781 (US)
(72) Inventor: NAUGHTON, Vincent, Libertyville, IL 60048 (US); HENRY, Jerome A., Libertyville, IL 60048 (US); MCMENAMIN, Martin, Libertyville, IL 60048 (US); GRIMES, Gerard P., Libertyville, IL 60048 (US); O'BRIEN, Daniel E., Libertyville, IL 60048 (US)
(74) Representative: FRKelly

(56) References cited:
- DE-T5- 112018 000 170
- US-A1- 2010 312 203

## Description

The present application claims the benefit of and priority to U.S. Provisional Application No. 62/939,848, filed November 25, 2019.

### TECHNICAL FIELD

The present disclosure generally relates to urinary catheters. More particularly, the present disclosure relates to urinary catheter collection bags.

### BACKGROUND

Catheters are used to treat many different types of medical conditions and typically include an elongated tube that is inserted into and through a passageway or lumen of the body. Catheters, and in particular intermittent catheters, are commonly used by those who suffer from various abnormalities of the urinary system, such as urinary incontinence. Urinary catheters generally comprise a tube with two ends. A first end has a catheter tip that may be inserted into a user's urethra. A second end generally has a drainage member that is used to help facilitate drainage of urine from the tube. Some urinary catheters include a urine collection bag connected to the drainage member of the catheter. When the bag is connected to the catheter, the catheter directs urine to flow into the attached collection bag.

After catheterization, the user typically empties the urine out of the bag into a toilet. The user then disposes the bag in a trash receptacle. DE112018000170 discloses a liquid waste bag having a tear part that is reasonably connected to an outer wall of a receptor cavity of the bag body which can be torn through so that liquid waste can be discharged out of the receptor cavity. Many catheter users have neurological conditions like stroke, Multiple Sclerosis (MS), or acute spinal cord injuries (SCI). Consequently, some catheter users may have limited dexterity, which in some instances can make it challenging for such users to open a urine collection bag. Accordingly, there is a need for urinary catheter collection bags that allow users to easily, cleanly and comfortably empty the bag themselves.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a urinary catheter collection bag comprises a first flexible wall having a perimeter, and a second flexible wall having a perimeter. A perimeter seal joins at least a portion of the perimeter of the first flexible wall to at least a portion of the perimeter of the second flexible wall, to define a cavity between the first and second flexible walls. The seal defines a boundary of the collection bag. A tear starting member extends beyond the boundary of the bag.

In another aspect, a surface of a urinary catheter collection bag includes a drain hole. The drain hole is covered by a water soluble cover member. The water soluble cover member is configured to dissolve after being submerged in a solution. After the water soluble cover member is dissolved, the drain hole is uncovered.

### Brief Description of the Drawings

Fig. 1 is a perspective view of an embodiment of a urinary catheter collection bag in accordance with the present disclosure;
Fig. 1A is a rear perspective view of the urinary catheter collection bag of Fig. 1;
Fig. 2 is a perspective view of the bag of Fig. 1 torn open and being emptied;
Fig. 3 is a perspective view of an embodiment of a urinary catheter collection bag in accordance with the present disclosure;
Fig. 4 is a perspective view of the bag of Fig. 3 with an exposed drain hole.

### Description of the Illustrated Embodiments

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

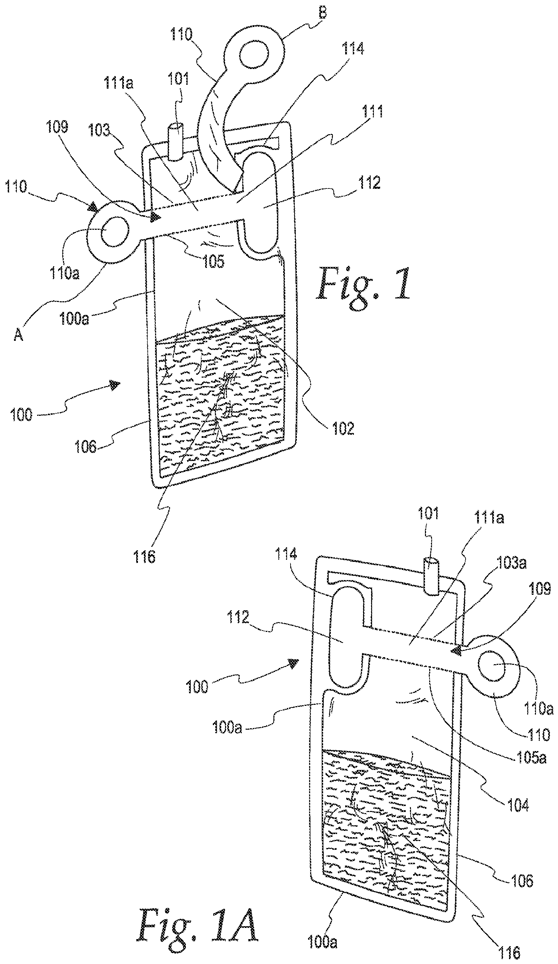

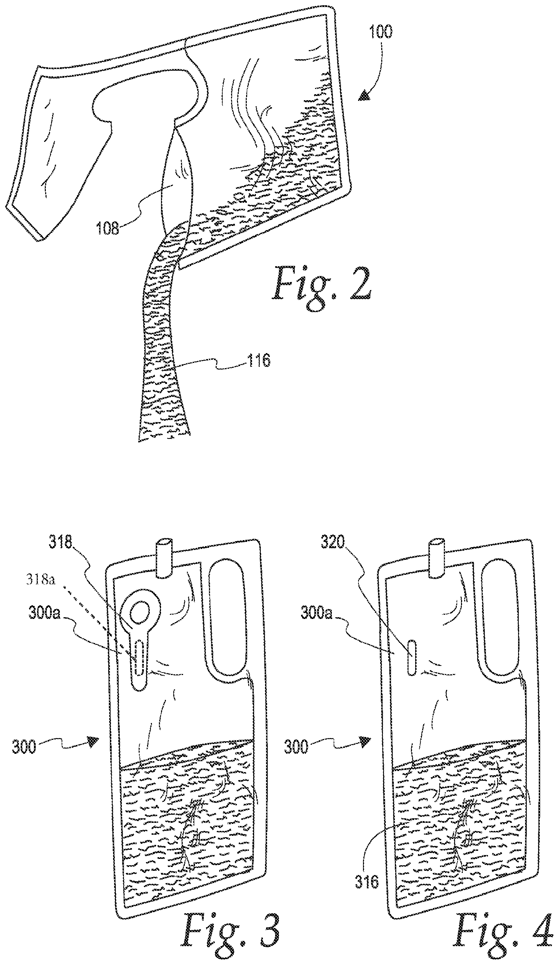

Urinary catheter collection bags according to the present disclosure and their individual components may be variously configured without departing from the scope of the present disclosure, but in one exemplary embodiment, a urinary catheter collection bag 100 is configured as shown in Figure 1. The collection bag 100 comprises a first flexible wall 102 having a perimeter, and a second flexible wall 104 (Fig. 1A) having a perimeter. A perimeter seal 106 joins at least a portion of the perimeter of the first flexible wall 102 to at least a portion of the perimeter of the second flexible wall 104 to define a cavity 108 (Fig. 2) between the first flexible wall 102 and second flexible wall 104. The seal 106 defines a boundary 100a of the collection bag 100. In the illustrated embodiment, the first flexible wall 102 and the second flexible wall 104 are defined by separate sheets of material that are sealed to one another. In another embodiment, the first and second walls 102 and 104 may be formed from a single sheet of material that is folded over to define the first and second walls. The walls 102 and 104 are connected at the fold located at bottom of the walls and are sealed together along the sides and tops of walls to form the collection bag 100. Additionally, the bag 100 includes a port or opening 101 for connection to a catheter. For example, the port 101 may be connected to a drainage member of a catheter.

The bag 100 also includes a tear starting member 110 that extends beyond the boundary 100a of the bag 100. The tear starting member 110 may be ring shaped and/or may include a hole 110a on an end that extends beyond the boundary 100a of the bag 100. In alternative embodiments, the tear starting member 110 may have any suitable shape and size.

As shown in Figs. 1 and 1A, in one embodiment the tear starting member 110 may extend from a tearable portion 109 located in the first and/or second walls 102 and 104 of the urine collection bag 100. In the illustrated embodiment, the tearable portion 109 may include tear strips 111 and 111a located in the first wall 102 and second wall 104, respectively. As shown in Fig. 1, the tear strip 111 may be defined by a first top tear line 103 and a second bottom tear line 105. Tear lines 103 and 105 extend across the first wall from the tear starting member 110 toward the opposite edge of the bag 100. Similarly and as shown in Fig. 1A, the tear strip 111a may be defined by a first top tear line 103a and a second bottom tear line 105a. Tear lines 103a and 105a extend across the second wall 104 from the tear starting member 110 toward the opposite edge of the bag 100. When the tearable portion 109 includes tear strips 111 and 111a, the tear strips may be opposed to one another. Furthermore, the tear starting member 110 may be integral or of unitary construction with the tearable portion 109. Alternatively, the tear starting member 110 may be attached to the tearable portion 109 by, for example, adhesive or welding.

Turning back to Fig. 1, this figure shows the tear starting member 110 and the tear portion 109 being moved from a first position A to a second position B to open the urine collection bag 100 for the drainage of urine therefrom. In use, the user grasps the tear starting member 110 and pulls it to tear the tear portion 109 of the collection bag. Tear lines 103 and 105 selectively direct tearing of the first flexible wall 102, and tear lines 103a and 105a selectively direct tearing of the second flexible wall 104. When the tear starting member 110 is in the second position B, it has traveled the full length of the first and second tear lines (103, 103a, 105, 105a) of each of the walls and is removable from the bag 100. When the starting member 110 is removed from the bag 100 the cavity 108 (shown in Fig. 2) is exposed and the bag's contents (the fluid within the bag) 116 may be emptied by the user. The tear lines 103, 103a, 105, and 105a may be score lines. In another embodiment, the tearable portion may be made from directional tear polymers, which direct tearing in a desired direction. For example, the directional tear polymers may direct tearing across the wall of the collection bag. In one embodiment, the tear lines of each wall are defined by directional tear polymers. In another embodiment tear lines 103, 103a, 105 and 105a may be defined by directional tear tape.

Additionally, the tear lines 103, 103a, 105, 105a may extend to a cutout 112 in the bag 100. The cutout 112 may comprise a terminus for the tear lines 103, 103a, 105, 105a. Additionally, the cutout 112 may form a handle 114 for gripping the bag 100.

The bag 100 may have a generally rectangular shape having a first side boundary and a second side boundary and the tear starting member 110 may extend from one of the side boundaries. Additionally, the first tear lines 103, 103a and the second tear lines 105, 105a, of the respective walls, may extend from one of the side boundaries toward the other of the side boundaries. For example, in an embodiment the first tear lines 103, 103a and second tear lines 105, 105a may both extend from the first side boundary to the second side boundary.

Fig. 2 shows the urinary catheter bag 100, after the tear starting member has been removed. As shown in Fig. 2, the bag 100 is open and the cavity 108 is exposed. A user may empty the bag 100 by draining the contents 116 through the cavity 108. The contents 116 may be drained into a toilet or any other appropriate receptacle.

Fig. 3 illustrates an embodiment of a urinary collection bag 300, which has similar structures to those described in Figs. 1 and 2. However, the urine collection bag 300 may be any other suitable bag for collecting urine. The bag 300 includes a surface 300a, where the surface of the bag 300a includes a drain hole 320 (visible in Fig. 4). As shown in Fig. 3, the drain hole is covered by a cover member 318a. In one embodiment, the cover member 318a is water soluble and may be attached to the bag 300 by an adhesive or may be integral to the bag 300. When made of a water soluble material, the cover member 318a is configured to dissolve after coming into contact with water or a solution containing water such that the drain hole is uncovered after the cover member is dissolved. Thus, when water touches the water soluble cover member the drain hole is exposed. Fig. 4 shows the bag 300 after the water soluble cover member 318a has dissolved and the drain hole 320 is exposed. When the drain hole 320 is exposed, the contents 316 of the bag 300 may exit through the drain hole 320 and into the toilet, or another appropriate receptacle. Thus a user may empty the bag 300, such that fluid within the bag (the contents) 316 may drain through the drain hole 320.

Fig. 3 also shows the water soluble cover member 318a situated behind a cover member 318. The water soluble cover member 318a dissolves, creating a drain hole. The covering member 318 may be made of materials including polyethylene (PE), or ethylene vinyl acetate (EVA). The covering member 318 sits in front of the water soluble cover member 318a. Once the water soluble cover member 318a is dissolved, the covering member 318 is removed to uncover the drain hole to allow the user to drain the bag in a toilet or appropriate receptacle. The water soluble cover member 318a may be made out of a water soluble material including, but not limited to, paper, polyvinyl alcohol (PVOH), polyvinyl acetate (PVA), or ethylene vinyl acetate (EVA).

In another embodiment, there is no water soluble cover member. Instead, the bag is pierced forming a drain hole. A self-adhesive cover member is placed above the drain hole in order to obstruct/stop the flow of urine out of the bag. The self-adhesive cover member is removed, uncovering the drain hole when the bag is ready to be drained in a toilet or other appropriate receptacle.

## Claims

1. A urinary catheter collection bag (300), comprising
a first flexible wall having a perimeter;
a second flexible wall having a perimeter;
a perimeter seal joining at least a portion of the perimeter of the first flexible wall and the perimeter of the second flexible wall;
the first flexible wall including a surface (300a);
a drainage hole (320) included in the surface (300a); and **characterised in that** the urinary catheter collection bag further comprises
a water soluble cover member (318a) covering the drainage hole (320), wherein the water soluble cover member (318a) is configured to dissolve after coming into contact with water, thereby exposing drainage hole (320).

2. The urinary catheter collection bag (300) of claim 1, further including a covering member in front of the water soluble cover member (318a).

3. The urinary catheter collection bag (300) of claim 2, wherein the covering member comprises polyethylene or ethylene vinyl acetate.

4. The urinary catheter collection bag (300) of any one of claims 1-3, wherein the water soluble cover member (318a) is attached to the surface (300a).

5. The urinary catheter collection bag of claim 4, wherein the water soluble cover member (318, 318a) is attached to the surface (300a) by an adhesive.

6. The urinary catheter collection bag (300) of any one of claims 1-3, wherein the water soluble cover member (318a) is integral with the bag (300).

7. The urinary catheter collection bag (300) of any one of claims 1-6, wherein the water soluble cover member is made from a water soluble material.

8. The urinary catheter collection bag (300) of claim 7, wherein the water soluble material comprises paper.

9. The urinary catheter collection bag (300) of claim 7, wherein the water soluble material comprises polyvinyl alcohol.

10. The urinary catheter collection bag (300) of claim 7, wherein the water soluble material comprises polyvinyl acetate.

11. The urinary catheter collection bag (300) of claim 7, wherein the water soluble material comprises ethylene vinyl acetate.

## Patentansprüche

1. Blasenkatheter-Sammelbeutel (300), umfassend
eine erste flexible Wand, die einen Umfang aufweist;
eine zweite flexible Wand, die einen Umfang aufweist;
eine Umfangsdichtung, die mindestens einen Abschnitt des Umfangs der ersten flexiblen Wand und des Umfangs der zweiten flexiblen Wand verbindet;
wobei die erste flexible Wand eine Fläche (300a) beinhaltet;
ein Entwässerungsloch (320), das in der Fläche (300a) beinhaltet ist; und **dadurch gekennzeichnet, dass** der Blasenkatheter-Sammelbeutel ferner
ein wasserlösliches Abdeckelement (318a) umfasst, das das Entwässerungsloch (320) abdeckt, wobei das wasserlösliche Abdeckelement (318a) dazu konfiguriert ist, sich aufzulösen, nachdem es mit Wasser in Kontakt gerät, wodurch das Entwässerungsloch (320) freigelegt wird.

2. Blasenkatheter-Sammelbeutel (300) nach Anspruch 1, der ferner ein Abdeckelement auf der Vorderseite des wasserlöslichen Abdeckelements (318a) beinhaltet.

3. Blasenkatheter-Sammelbeutel (300) nach Anspruch 2, wobei das Abdeckelement Polyethylen oder Ethylenvinylacetat umfasst.

4. Blasenkatheter-Sammelbeutel (300) nach einem der Ansprüche 1-3, wobei das wasserlösliche Abdeckelement (318a) an der Fläche (300a) befestigt ist.

5. Blasenkatheter-Sammelbeutel nach Anspruch 4, wobei das wasserlösliche Abdeckelement (318, 318a) durch ein Haftmittel an der Fläche (300a) befestigt ist.

6. Blasenkatheter-Sammelbeutel (300) nach einem der Ansprüche 1-3, wobei das wasserlösliche Abdeckelement (318a) einstückig mit dem Beutel (300) ist.

7. Blasenkatheter-Sammelbeutel (300) nach einem der Ansprüche 1-6, wobei das wasserlösliche Abdeckelement aus einem wasserlöslichen Material hergestellt ist.

8. Blasenkatheter-Sammelbeutel (300) nach Anspruch 7, wobei das wasserlösliche Material Papier umfasst.

9. Blasenkatheter-Sammelbeutel (300) nach Anspruch 7, wobei das wasserlösliche Material Polyvinylalkohol umfasst.

10. Blasenkatheter-Sammelbeutel (300) nach Anspruch 7, wobei das wasserlösliche Material Polyvinylacetat umfasst.

11. Blasenkatheter-Sammelbeutel (300) nach Anspruch 7, wobei das wasserlösliche Material Ethylenvinylacetat umfasst.

## Revendications

1. Sac de collecte pour cathéter urinaire (300), comprenant
une première paroi souple présentant un périmètre ;
une seconde paroi souple présentant un périmètre ;
un joint périphérique reliant au moins une partie du périmètre de la première paroi flexible et le périmètre de la seconde paroi flexible ;
la première paroi flexible comportant une surface (300a) ;
un orifice d'évacuation (320) compris dans la surface (300a) ;
et **caractérisé en ce que** le sac de collecte de cathéter urinaire comprend également
un élément de couvercle hydrosoluble (318a) recouvrant l'orifice d'évacuation (320), dans lequel l'élément de couvercle hydrosoluble (318a) est configuré pour se dissoudre après être entré en contact avec l'eau, exposant ainsi l'orifice d'évacuation (320).

2. Sac de collecte de cathéter urinaire (300) selon la revendication 1, comprenant également un élément de recouvrement devant l'élément de couvercle hydrosoluble (318a).

3. Sac de collecte de cathéter urinaire (300) selon la revendication 2, dans lequel l'élément de recouvrement comprend du polyéthylène ou de l'éthylène-acétate de vinyle.

4. Sac de collecte de cathéter urinaire (300) selon l'une quelconque des revendications 1 à 3, dans lequel l'élément de couvercle hydrosoluble (318a) est fixé à la surface (300a).

5. Sac de collecte de cathéter urinaire selon la revendication 4, dans lequel l'élément de couvercle hydrosoluble (318, 318a) est fixé à la surface (300a) par un adhésif.

6. Sac de collecte de cathéter urinaire (300) selon l'une quelconque des revendications 1 à 3, dans lequel l'élément de couvercle hydrosoluble (318a) fait partie intégrante du sac (300) .

7. Sac de collecte de cathéter urinaire (300) selon l'une quelconque des revendications 1 à 6, dans lequel l'élément de couvercle hydrosoluble est fabriqué à partir d'un matériau hydrosoluble.

8. Sac de collecte de cathéter urinaire (300) selon la revendication 7, dans lequel le matériau hydrosoluble comprend du papier.

9. Sac de collecte de cathéter urinaire (300) selon la revendication 7, dans lequel le matériau hydrosoluble comprend de l'alcool polyvinylique.

10. Sac de collecte de cathéter urinaire (300) selon la revendication 7, dans lequel le matériau hydrosoluble comprend de l'acétate de polyvinyle.

11. Sac de collecte de cathéter urinaire (300) selon la revendication 7, dans lequel le matériau hydrosoluble comprend de l'éthylène-acétate de vinyle.
